# EUROPEAN PATENT SPECIFICATION

(11) **EP 3 055 412 B1**
(45) Date of publication and mention of the grant of the patent: **02.09.2020**
(21) Application number: 14781529.4
(22) Date of filing: 07.10.2014
(51) Int. Cl.: C12N 7/02

(54) **TREATED FILTER**
BEHANDELTER FILTER
FILTRE TRAITÉ

(30) Priority: 07.10.2013 EP 13187628
(43) Date of publication of application: 17.08.2016
(73) Proprietor: Seqirus UK Limited, Maidenhead, Berkshire SL6 8AA (GB)
(72) Inventor: CHAN, Gerard, Liverpool Merseyside L24 9GR (GB); MURPHY, Kevin, Liverpool Merseyside L24 9GR (GB); SIZER, Philip J., Liverpool Merseyside L24 9GR (GB); NEUMANN, Arne, 39179 Barleben (DE); ROTH, Bernhard, 35041 Marburg (DE)
(74) Representative: Carpmaels & Ransford LLP
(86) International application number: PCT/EP2014/071426
(87) International publication number: WO 2015/052177

(56) References cited:
- WO-A1-00/13764
- WO-A1-2007/133762
- WO-A1-2011/051235
- WO-A1-2013/127709
- YANG S ET AL: "Loading characteristics of filter pretreated with anionic surfactant for monodisperse solid particles", POWDER TECHNOLOGY, ELSEVIER SEQUOIA, LAUSANNE, CH, vol. 156, no. 1, 2 August 2005 (2005-08-02), pages 52-60, XP027793505, ISSN: 0032-5910 [retrieved on 2005-08-02]

## Description

### FIELD OF THE INVENTION

The present invention relates to a method of filtering fluid comprising proteins. The present invention also relates to a treated filter for use in the method and the filtrate resulting from the method.

### BACKGROUND OF THE INVENTION

During the processing of proteinaceous matter it is commonly required to apply a filtration step. Such a filtration step enables the removal of components that would be detrimental to the final product. For example, by choosing a filter with a suitable pore size, it is possible to separate free, whole virus particles from large aggregates of virus particles.

EP0188104 81 describes filtration of blood products through a device such as a cardiotomy reservoir containing a filter. The filter has been treated with polysorbate 80 which improves the gravitational flow of blood through the device. However, the technique is limited to blood filtration and does not disclose purification of therapeutic protein, like viral proteins or virions for vaccines. EP0188104 does not use the filtration to remove large protein aggregates from the protein of interest. Furthermore, EP0188104 requires drying of the filters prior to use which is not necessary under the present invention.

WO/2011/051235 described the addition of t-octylphenoxypolyethoxyethanol (TRITON X-100®) to increase filtration performance in flu vaccine manufacturing. However, in WO/2011/051235 Triton is added to the virus preparation and not used to pre-treat the filter. Furthermore, in WO/2011/051235 is added after splitting and before inactivation. In the present case the use of detergent-treated filters before splitting is preferred.

### SUMMARY OF THE INVENTION

In the manufacturing of viral products, filtration of proteinaceous matter, product sterility and safety is key. Inactivation steps are taken to kill infectious matter. Commonly, inactivating agents, such as formaldehyde, kill the infectious replication machinery by inducing crosslinking of proteinaceous matter, aggregated proteins can also be produced. It has been found that the filtration of a fluid comprising proteinaceous matter can be remarkably inefficient. For example, a substantial reduction in the amount of desirable product has been observed despite filtration and purification steps, and even when the particle size of the proteinaceous matter is such that it would be expected to easily pass through the filter.

The present inventors have determined that such unintended loss of biological products of interest (e.g., proteinaceous matter) can be avoided by treating the filter prior to, or during filtration of fluid comprising the proteinaceous matter. In particular, it has been unexpectedly discovered that pretreatment of membrane filters with a surfactant can result in a surprising improvement in the yield of recovery of biological products of interest, as compared to control, i.e., non-pretreated membrane filter that is otherwise identical used under otherwise equivalent condition(s).

Accordingly, the present invention provides a method of purifying biological products, such as proteins, from a mixture which include large aggregate impurities. The methods of the present invention are defined in claims 1, 2 and 4. According to the invention, the biological product is filtered through a suitable filter membrane having a size that retains the large protein aggregate impurities. The hydrophobic filter is pre-treated with a surfactant which decreases the binding of the biological product of interest to the filter membrane.

Biological products which can be filtered by the present invention include antibodies, blood products and viral proteins.

### BRIEF DESCRIPTION OF THE FIGURES

Figure 1 illustrates the percentage protein loss for various strains of the influenza virus after filtration through an untreated filter.
Figure 2 illustrates the change in flowrate with time for various strains of the influenza virus being passed through an untreated filter.
Figure 3 illustrates the particle size distribution of a strain of the influenza virus before and after filtration with an untreated filter.
Figure 4 illustrates the increase in yield of recovered virus caused by treating the filterin accordance with the present invention relative to filtering with an untreated filter.
Figure 5 illustrates the amount of polysorbate 80 (Tween) removed with varying phosphate buffered saline flush volume.
Figure 6 illustrates the percentage loss of virus when filtering with a filter treated with a phosphate buffered saline solution relative to a filter treated with polysorbate 80 (Tween).

### DESCRIPTION OF CERTAIN EMBODIMENTS OF THE INVENTION

In one aspect, the invention relates to a method for purifying a biological product of interest from impurities by large protein aggregates. Accordingly, methods for removing aggregates from a sample (e.g., fluids) containing at least one biological product of interest are provided.

According to the invention, the method involves the use of an appropriate membrane filter which has been pre-treated with a surfactant, e.g., a surfactant solution, so as to provide a pretreated membrane filter. As described in further detail herein, the pretreated membranefilter is typically not dried following the step. The pretreated membrane filter may be optionally washed with a suitable buffer or water to remove excess (e.g., non-bound) surfactant from the membrane filter prior to a filtration step. Once a pretreated membrane filter is obtained, and optionally washed, the pretreated membrane filter is then used to filter a sample (e.g., fluid samples) containing a biological product of interest. The biological product of interest is filtered through the pretreated membrane filter and is collected as a filtrate. This step removes unwanted large aggregates from the sample.

In some embodiments, methods for preventing loss of a biological product during filtration are provided. Reducing unwanted loss of products during manufacture is an important factor especially for biologic products. Relative yield of recovery with respect to filtration can be determined by comparing the initial amount of a biological product present in a sample prior to a filtration step and the final amount of the biological product present in a filtrate after the filtration step. It is desirable that yield is as close to 100% as possible, e.g., about 100%, at least 95%, at least 90%, at least 85%, at least 80%, at least 75%, at least 70%, at least 65%, at least 60%, etc.

Thus, the invention provides methods in which filtering a fluid comprising a first amount of a biological product through the pretreated membrane filter so as to recover the biological product in a filtrate, wherein the filtrate comprises a second amount of the biological product. In some embodiments, the second amount of the biological product is at least 75% of the first amount of the biological product, e.g., at least 80%, at least 85%, at least 90%, at least 95%.

Typically, in any of the embodiments of the invention, suitable membrane filters include hydrophobic membrane filters. In addition, suitable membrane filters are selected on the basis of pore size in order to filter through a biological product of interest while retaining aggregates which may be present in the fluid sample. As shown in data presented herein, a significantly higher yield of recovery of the biological product in the filtrate can be achieved using the methods described herein, as compared to a non-pretreated control.

The invention is useful for processing of compositions comprising one or more biological products, particularly for compositions which require high purity. Thus, the invention is useful for the production of pharmaceutical, nutraceutical and/or cosmetic products. In some embodiments, the biological product is or comprises a protein, a virus-like particle, a viral particle, a virion, or any combination thereof.

In some embodiments, the biological product is filtered through a hydrophobic filter membrane with a pore size that retains the large protein aggregates impurities which are not products of interest, whereby the filter membrane has been pre-treated with a detergent, and the pre-treatment decreases the binding of the biological product of interest to the filter membrane. The method might include further steps, in particular the formulation of the biological product of interest in a form suitable for administration to a human or animal patient.

In a preferred embodiment the biological product of interest is a protein. Large protein aggregates are to be determined relative to the size/weight of the biological product of interest. Large protein aggregates are understood to be substantially larger in size and/or weight than the biological product of interest. Substantially larger means that the size and/or the weight is at least 8 times, preferably 10, 15, 20, 30, 50 times higher than the product of interest. Size and weight of a protein aggregate can be determined e.g. by gel electrophoresis. If viruses are the products of interest, the size of the large aggregates is at least 1µm, preferably ≥1.5; ≥2; ≥3; ≥4; ≥5; or ≥10 µm. If a virus protein, e.g. the HA or NA antigen is the product of interest, the size of the large aggregates is at least 0.2 µm, preferably ≥0.5; ≥0.8; ≥1, ≥2; ≥3; ≥4; ≥5; or ≥10 µm. If the product of the filtration is different in size/weight from the final (therapeutic) product, e.g. because the filtered product is further processed by splitting, cleavage, tagging, etc., then 'product of interest' means the product of the filtration step and not the final (processed) product.

In one embodiment the large protein aggregates are connected by covalent bonds, e.g. through crosslinking by formaldehyde. In another embodiment the large aggregates are connected through non-covalent bonds (e.g. ionic or hydrophobic bonds). The protein aggregates might contain non protein components such as nucleic acids or carbohydrates. In a preferred embodiment the biological product of interest binds strongly to the filter material if the filter material has not been pre-treated as described by the present invention. This binding might even be irreversible under conditions which would retain integrity and/or biological function of the product of interest.

Accordingly, one aspect of a disclosure not recited in the claims provides a method of filtering a fluid through a filter, wherein the fluid comprises a viral protein, particularly an influenza antigen and the method comprises the steps of (i) contacting the filter with a solution comprising a surfactant to produce a treated filter; and (ii) passing the fluid through the treated filter to give a filtrate.

In the present invention, step (i) is carried out before step (ii). Alternatively, in a disclosure not recited in the claims, step (i) and step (ii) can be carried out simultaneously, as described below.

In another aspect, there is provided a method of filtering a fluid through a filter, wherein the fluid comprises a virus or viral protein and the filter has been contacted with a solution which is different from the virus preparation comprising a surfactant to produce a treated filter and the method comprises the step of passing the fluid through the treated filter to give a filtrate. The filtrates produced may be subjected to further processing such as chromatographic purification, depth-filtration, and/or sterilization to produce a vaccine formulation.

Another approach would be to prepare a viral protein cultivated from eggs or cell culture. Manufacturing techniques that can be used include growing virus in eggs in culture or cell culture, clarifying the mixture containing the virus to remove debris, inactivating the mixture chemically or physically, filtering the inactivated virus through a filter which has been treated with a surfactant then further processing of the virus into a vaccine. Based on the findings presented herein, a person skilled in the art will be able to either prepare a sample for filtering on the treated filter or filter a sample which has been clarified, inactivated, filtered, and/or split using the method of the invention to produce an immunogenic protein for formulation into a vaccine.

Accordingly, the present invention provides a method of filtering a fluid through a filter, wherein the fluid comprises a viral protein and the method comprises the steps of providing a sample comprising influenza virus grown in eggs or in a cell culture; inactivating the virus with an inactivating agent to provide an inactivated virus; filtering the inactivated virus through a hydrophobic filter whereby the filter has been treated with a surfactant; splitting the virus, processing the inactivated virus into a vaccine. In this embodiment it is preferred that the filtration occurs prior to the splitting because the large aggregates decrease splitting efficiency.

In an alternative embodiment the present invention provides a method of filtering a fluid through a filter, wherein the fluid comprises a viral protein and the method comprises the steps of (i) providing a sample comprising influenza virus grown in eggs or in a cell culture; (ii) splitting the virus (iii) filtering the split virus through a hydrophobic filter whereby the filter has been treated with a surfactant (iv) optionally inactivating the virus; (v) optionally formulating the virus antigens into avaccine.

In an alternative embodiment the present invention provides a method of filtering a fluid through a filter, wherein the fluid comprises a viral protein and the method comprises the steps of (i) providing a sample comprising influenza virus grown in eggs or in a cell culture; (ii) splitting the virus; (iii) inactivating the virus; (iv) filtering the split virus through a hydrophobic filter whereby the filter has been treated with a surfactant; (iv) optionally formulating the viral proteins into a vaccine.

In a related aspect, the present invention provides a method further comprising purifying the virus prior to or after the filtering steps. Purification of viral particles and proteins can be accomplished using optimal technology such as depth filtration, the choice of which is known to the skilled artisan guided by the properties of the starting material and the required filtrate quality. Suitable depth filtration includes tangential flow filtration, ultrafiltration, crossflow filtration, diafiltration, etc.

In another related aspect the method of the present invention further comprises splitting the virus with a splitting agent prior to the filtering step. Suitable splitting agents known in the art may be used, such as beta propiolactone (BPL) or cetyltrimethyl ammonimum bromide (CTAB).

In yet another aspect, the present method provides flushing the treated filter to remove at least a portion of the unbound surfactant prior to passing the fluid through the treated filter to give the filtrate.

The fluid may be any medium that is suitable for carrying a protein through the filter. An example of such a suitable fluid is water, or a fluid that comprises water. Suitable fluids include aqueous buffers, such as Phosphate buffered saline, Citric acid, Acetic acid, K2HPO4, CHES, Borate, TAPS, Bicine, Tris, Tricine, TAPSO, HEPES, TES, MOPS, PIPES, Cacodylate, SSC, MES, or Succinic acid. The fluid may have a pH of between 5.0 and 8.1, and more typically between 6.0 and 8.0 e.g. 6.5 and 7.5, or between 7.0 and 7.8.

A protein present in the fluid may be a viral protein. It is particularly preferred that the protein is an influenza virus protein, particularly a surface glycoprotein.

The protein in the fluid can be in soluble virion form, or can be a subvirion particle. Thus an influenza virus protein, such as hemagglutinin, can be present in solution or as part of an influenza virion. Thus the fluid can include more than one protein (e.g. hemagglutinin and neuraminidase), either in whole virion or subvirion form.

The filter may comprise a polymeric, hydrophobic material. In particular, the filter may comprise at least one of polypropylene, polyvinylidene chloride, cuprammonium regenerated cellulose, polyvinylidene fluoride (PVDF), polysulfone, polyethersulfone, nylon, polyester, and PEEK. The filter is preferably a polypropylene filter, since the invention has been found to be particularly effective with polypropylene filters. A preferred filter is SARTOBIND PP2 depth filter which comprises multiple poly-propylene layers and various pore sizes ranging from 0.65 microns to 50 microns.

The filter is preferably woven from fibres to form a layer which selectively retains molecules by their size. Alternative filters which may be used include gels or other resin which selectively retain molecules in solution by size and/or charge. Multiple filters can be used sequentially or simultaneously depending on the sample characteristics.

The filter is contacted with a solution comprising a surfactant to produce a treated filter. It is preferable to maintain the filter in a wetted statebefore filtering biological products. It is therefore preferred that the filter is not dried before use.. In a preferred embodiment a detergent is used which already utilized within the purification process so that there is less concern with its removal from the further downstream processing.

The surfactant is preferably a non-ionic surfactant. The surfactant is preferably a hydrophilic surfactant. Preferred surfactants of the invention have a HLB (hydrophile/lipophile balance) of at least 10, preferably at least 15, and more preferably at least 16. The surfactant may be selected from Cetomacrogol 1000, Cetostearyl alcohol, Cetyl alcohol, Cocamide DEA, Cocamide MEA, Decyl glucoside, IGEPAL CA-630, Isoceteth-20, Lauryl glucoside, Monolaurin, Narrow range ethoxylate, Nonidet P-40, Nonoxynol-9, Nonoxynols, NP-40, Octaethylene glycol monododecyl ether, Octyl glucoside, Oleyl alcohol, Pentaethylene glycol monododecyl ether, Poloxamer, Poloxamer 407, Polyglycerol polyricinoleate, Polysorbate, Sorbitan monostearate, Sorbitan tristearate, Stearyl alcohol, and Triton X-100. In one aspect, the surfactants chosen should reduce hydrophobic interactions and bind irreversibly to the filter and yet does not split or disrupt the proteins or its immunogenicity. In a preferred embodiment, the detergent used to treat the filter is different from those used as splitting agents.

It is particularly preferred that the surfactant is a polysorbate. Possible polysorbates are polysorbate 20, polysorbate 40, polysorbate 60 and polysorbate 80. A particularly preferred form of polysorbate is polysorbate 80, which is also known as Tween 80. However, it is also preferred to use detergents with similar properties as Tween, e.g. with a similar high HLB value.

The mass concentration of the surfactant in the solution may be in the range of 0.1 to 10 % w/v surfactant, alternatively 0.1 to 5 %w/v surfactant, alternatively 0.1 to 2 %w/v, or 0.01 to 1.0 %w/v (for example 0.1, 0.2, 0.3, 0.4, 0.5, 0.6, 0.7, 0.8, 0.9 and 1.0 %w/v). Surfactant concentration can include those amounts above the critical micellar concentration of the surfactant, for example by reference, greater than 0.0016% w/v for Polysorbate 80. Preferably, the surfactant solution has a mass concentration of 0.6 %w/v surfactant, since this amount has been found to be particularly effective.

This treatment of the filter can occur prior to the protein contacting the filter. In this way, the protein only contacts a treated filter and the beneficial effect of the pre-treatment is utilised with all of the filtered fluid. However, it is also possible that the polysorbate 80 is passed through the filter with the fluid being filtered. This latter approach still results in the beneficial effects of the present invention.

The fluid comprising the protein is passed through the treated filter to produce a resulting filtrate. The filtrate is characterised by lower loss of proteinaceous matter of interest relative to a filtrate produced when using a filter that has not been treated in accordance with the present invention. The treated filter increases the yield of desired product in the pass through relative to an untreated filter. Without wishing to be bound by theory, it is believed that the treatment of the filter with a solution comprising a surfactant inhibits viral proteins and the selected virions from binding to the filter during the filtration process. This binding might be strong and even irreversible under conditions which would retain integrity and antigenicity of the viral proteins of interests. Without treatment of the filter according to the present invention, the amount of protein, particularly virion, that can selectively pass through the filter, and so be present in the resulting filtrate, is significantly reduced.

After treating the filter, and before filtering the preparation of interest, at least a proportion of unbound surfactant may be removed by flushing the filter with a liquid.

Flushing may be achieved using a buffer solution. The buffer solution may be Phosphate buffered saline, Citric acid, Acetic acid, K2HPO4, CHES, Borate, TAPS, Bicine, Tris, Tricine, TAPSO, HEPES, TES, MOPS, PIPES, Cacodylate, SSC, MES, or Succinic acid. It is preferred that the buffer solution is phosphate buffered saline. This step removes at least some of the surfactant but still results in a treated filter that demonstrates the advantageous properties described herein.

A treated filter is a filter that has been contacted by a solution containing a surfactant described herein. Such a treated filter results in a substantial reduction in the amount of proteinaceous matter of interest that does not pass through the treated filter relative to an untreated filter.

The pore size of the filter used with the present invention may be in the range of 0.1 microns to 10 microns (as used herein microns refers to micrometres), alternatively in the range of 1 micron to 6 microns (for example, 1.0, 1.5, 2.0, 2.5, 3.0, 3.5, 4.0, 4.5, 5.0, 5.5, and 6.0 microns). The present invention has been found to be particularly effective with filters of this pore size.

When assessing the suitability of a filter for separation capacity, it is desirable to know the size of aggregates that may form and could be withheld by the filter. In a particular aspect, an influenza virus is known to have an individual particle size of 120-180 nm. It is thus preferable to use a filter that sufficiently withholds aggregate impurities but allows passage of the desired product, such as whole individual virus particles, hemagglutinin and neuraminidase proteins. For purification of whole virus from aggregates, at least a 1.0 micron, 2.0 micron, 3.0 micron, 4.0 micron, 5.0 micron filters are utilized.

Preferably, the filters are utilized in depth-filtration techniques described herein.

As noted above, the present invention has been found to be particularly effective with influenza virus proteins. Therefore, the protein in the fluid may be an influenza surface protein. The influenza surface protein may have been isolated from the whole virus or it may be present in the form of the surface protein in situ as part of the whole influenza virus. The influenza virus can be an influenza A virus or an influenza B virus. Where it is an influenza A virus it may have any of influenza A virus HA subtypes H1, H2, H3, H4, H5, H6, H7, H8, H9, H10, H11, H12, H13, H14, H15 or H16, and any of influenza A virus NA subtypes N1, N2, N3, N4, N5, N6, N7, N8 or N9. Where it is an influenza B virus, it can be a B/Victoria/2/87 like or B/Yamagata/16/88 like strain.

The influenza viruses can be propagated using a cell line, although primary cells may be used as an alternative. The cell will typically be mammalian, although avian or insect cells can also be used. Suitable mammalian cells include, but are not limited to, human, hamster, cattle, primate and dog cells. In some embodiments, the cell is a human non-kidney cell or a non-human cell. Various cells may be used, such as kidney cells, fibroblasts, retinal cells, lung cells, etc. Examples of suitable hamster cells are the cell lines having the names BHK21 or HKCC. Suitable monkey cells are e.g. African green monkey cells, such as kidney cells as in the Vero cell line. Suitable dog cells are e.g. kidney cells, as in the CLDK and MDCK cell lines. Suitable avian cells include the EBx cell line derived from chicken embryonic stem cells, EB45, EB14, and EB14-074.

The influenza viruses prepared according to the methods of the invention may be cultured in eggs as described in US1498261. Basically, allantoic fluid containing the virus is pooled and the virus concentrated and purified by centrifugation by sucrose gradient in phosphate buffered saline. The virus is further concentrated by dialysis in phosphate buffered saline and inactivated. The HA and NA proteins are split off the virus by reaction with CTAB then worked up by gradient centrifugation or molecular sieve chromatoraphy to separate the virus residual particle from the proteins. Thus part of the present invention is a process for preparing an influenza vaccine in which the following steps are conducted: (1) virus is cultured in eggs (2) the allantoic fluid containing the virus is harvested; (3) the virus is concentrated and purified (4) the virus is inactivated; (5) the virus is filtered through membrane pre-treated with a detergent; (6) the virus is split by treatment with CTAB; (7) the virus proteins of interest are purified, and (8) optionally the viral proteins are formulated into a vaccine.

US20110243987 provides detailed description of suitable preparation of viral proteins. Basically, the manufacturing process can be divided in four main parts: 1) propagation of the working seed in fertilized hen's eggs, harvesting and pooling of infected allantoic fluids so as to obtain the "crude monovalent whole virus bulk". 2) purification of each virus strain leading to the "purified monovalent whole virus bulk". 3) splitting of the purified monovalent whole virus bulk with sodium deoxycholate resulting in the "purified monovalent split virus bulk". 4) inactivation of the purified monovalent split virus bulk in two steps by incubation with sodium deoxycholate and with formaldehyde, followed by ultrafiltration and sterile filtration, in order to obtain the "purified monovalent inactivated split virus bulk" or "Monovalent Bulk"

The production of the purified monovalent whole virus bulk is as follows: The harvested allantoic fluid is clarified by continuous moderate speed centrifugation. This step removes big particles that could have been collected during the harvest of the allantoic fluid (e.g. parts of egg shells). Adsorption Step: This step permits further clarification of the allantoic fluid through precipitation of virus material, by adsorption to a dibasic calcium hydrogen phosphate gel. After sedimentation for at least 8 hours to a maximum 36 hours, the supernatant is removed and the sediment containing the influenza viruses is re-solubilized in 8.7% disodium EDTA solution. Filtration: The resuspended influenza sediment is filtered through a 6-µm filter membrane to remove potential remaining pellets.

Flow through Ultracentrifugation: The influenza virus is further purified (removal of proteins and phospholipids) and concentrated by isopycnic ultracentrifugation in a linear sucrose gradient. The virus containing fractions are further purified ultrafiltration. The production of the purified monovalent split virus bulk is as follows: The influenza virus is split and further purified by centrifugation through a linear sucrose gradient that contains 1.5% sodium deoxycholate. Tween-80 is present at 0.1% in the gradient.

The preparation of the purified final monovalent split, inactivated virus bulk is as follows: The purified monovalent split virus bulk is gradually filtered down to a 0.45 µm filter membrane, briefly sonicated (to facilitate filtration) and filtered through a 0.2 µm membrane. At the end of the filtration, the filters are rinsed with phosphate buffer containing 0.025% Tween-80. Sodium deoxycholate Inactivation: The resulting solution is incubated at 22 ± 2°C for at least 84 hours. After completion of the first inactivation step, the material is diluted with phosphate buffer to reduce the total protein content to a calculated concentration of 500 µg/mL:

Formaldehyde Inactivation: Formaldehyde is added to a calculated final concentration of 100 µg/mL. Inactivation takes place in a single use low density polyethylene 100 L bag at 20 ± 2°C for at least 72 hours. The inactivated split virus material is ultrafiltered through membranes with a molecular weight cut off of 30,000 Dalton. After a volume reduction, the volume remains constant during ultrafiltration (diafiltration) by adding phosphate buffer and phosphate buffered saline containing 0.01%Tween-80. During ultrafiltration, the content of formaldehyde, NaDoc and sucrose is reduced. The material concentrated to 15 - 25 liters and is transferred immediately to the final filtration step. Sterile Filtration: After ultrafiltration, the split inactivated material is gradually filtered down to a 0.2 µm membrane. The final sterile filtration through a 0.22 µm sterile grade membrane.

The present invention can be applied on different steps during the process described in US20110243987. One embodiment of the present invention is a method for producing a purified monovalent whole virus bulk is which the following steps are conducted: (1) an influenza virus is cultured in eggs; (2) the allantoic fluid is harvested and clarified ; (3) allantoic fluid containing the virus is pooled; (3) the virus is precipitated by adsorption to a dibasic calcium hydrogen phosphate gel; (4) the virus is resuspended; (5) the virus is filtered through a membrane, whereby the membrane has been pre-treated with a detergent.

Another embodiment is the production of a purified monovalent split virus bulk in which the following steps are conducted: (1) a purified monovalent split virus bulk is split (preferably with sodium deoxycholate); (2) the split virus is purified (preferably by centrifugation); (3) the purified split virus is filtered through a filter membrane which has been pre-treated with a detergent. Preferably the filter membranes are a 0.45 µm filter membrane followed by a 0.2 µm membrane.

Another embodiment of the present invention is the production of an inactivated purified monovalent inactivated split virus bulk, wherein the following steps are conducted: (1) a purified monovalent split virus bulk is inactivated; (2) the inactivated purified monovalent bulk is filtered through a filter membrane which has been pre-treated with a detergent. In a preferred embodiment the inactivation is done with Sodium deoxycholate and Formaldehyde.

The present invention has also been found to be particularly effective with the combination of polysorbate 80 and a polypropylene filter. This combination is especially effective with fluids containing the influenza virus and subvirion particle.

The method of the present invention may involve passing the fluid comprising a protein through more than one filter in order to obtain the filtrate. If more than one filter is used, at least one of the filters is a treated filter. Preferably all of the filters used to produce the filtrate are treated filters. For example, the fluid could be passed through two treated filters. Each of the treated filters employed with the method can have the same pore size as each other. As an alternative, one or more of the filters may have a different pore size to the other filters employed in the method. In one possible arrangement, the fluid is passed through a treated filter with a pore size of 5 µm and then though a treated filter with a pore size of 1.2 µm in order to produce the filtrate.

The method of the present invention may comprise at least one further purification step. Where the protein is present as part of a whole virus, the method of the present invention may further comprise the step of inactivating the virus. The method of the present invention may also comprise additional processing steps, for example at least one further purification step prior to or after the step of inactivating the virus. The person of skill in the art will be able to apply the optimal processing steps needed for purification of protein and viruses.

In one aspect, the present invention relates to a method for producing an immunogenic composition comprising the step of filtering the fluid as described herein and preparing an immunogenic composition from the filtrate. The immunogenic composition is preferably an influenza vaccine, e.g. a trivalent vaccine, a monovalent vaccine, a tetravalent vaccine or a heptavalent vaccine.

Vaccines (particularly for influenza virus) are generally based either on live virus or on inactivated virus. Inactivated vaccines may be based on whole virions, 'split' virions, or on purified surface antigens. Antigens can also be presented in the form of virosomes. The invention can be used for manufacturing any of these types of vaccine. Where an inactivated virus is used, the vaccine may comprise whole virion, split virion, or purified surface antigens (for influenza, including hemagglutinin and, usually, also including neuraminidase).

The methods of the invention may also be used to produce live vaccines. Such vaccines are usually prepared by purifying virions from virus-containing fluids.

Another aspect of the present invention relates to a method for producing an immunogenic composition using the filtrate produced by a method described herein.

In one aspect, a disclosure not recited in the claims relates to a filtrate produced by a method of the present invention. The filtrate is characterized in having a low amount of protein loss in the particle size range that should readily pass through the filter. In other words, the filtrate of the present disclosure has much lower protein losses when compared to a filtrate of the prior art.

The amount of protein present in the filtrate that, due to its size relative to the filter pore size, would be expected to pass through the filter may be less than 10% lower than the amount of protein present in the pre-filtration fluid that, due to its size relative to the filter pore size, would be expected to pass through the filter. It may be less than 8% lower or preferably less than 5%, less than 4%, less than 3% less than 2% or less than 1% lower.

The resulting increase in yield of protein in the filtrate of the present disclosure relative to the filtrate of an untreated filter may be 10% or greater more, 15% or greater more or, preferably, 20% greater or more, 30% greater or more, 40% greater or more, 50% greater or more, 60% greater or more, 70% greater or more, 80% greater or more, 90% greater or more.

The amount of protein present in the filtrate can be measured using any of the methods known in the art, for example the amount of protein present can be measured using the BCA assay for the protein. If the protein is in the form of an influenza virus, it can also be measured by quantitative PCR assay specific for influenza virus RNA.

In another aspect, a disclosure not recited in the claims relates to a treated filter for use in the method of the present invention. In particular the present disclosure relates to the use of pre-treated filters in vaccine manufacturing. The filter is treated with a solution comprising a surfactant such that the tendency for proteinaceous matter to bind to the filter is reduced. The filter may be treated as described hereinabove. The filter may have a composition as described hereinabove.

This invention is further illustrated by the following examples which should not be construed as limiting.

### EXAMPLES

Filtration was carried out on a selection of purified solutions of formaldehyde-inactivated whole influenza viruses. Such a step is utilised in the processing of influenza viruses to filter out any large virus complexes or aggregates before the virus is solubilized through the addition of Tween 80 and cetyl triammonium bromide (CTAB) surfactants.

Although influenza virus has a pleiomorphic structure, a reasonable estimate for its average diameter is around 120 nm (0.12 microns). The clarifying filtration utilised was a dual-stage process through two polypropylene filters; the first filter having a pore size of 5 microns, the second filter having a nominal pore size of 1.2 microns. Free, individual virus particles should therefore easily pass through these filters, while large aggregates that may haveformed during upstream processing will be with-held by the filter. It is important that any such aggregates are removed from the process as they will not be effectively solubilized during the subsequent virus splitting stage. The number of large aggregates, as a proportion of the total filtered material, is predicted to very low but may be influenza virus strain-specific.

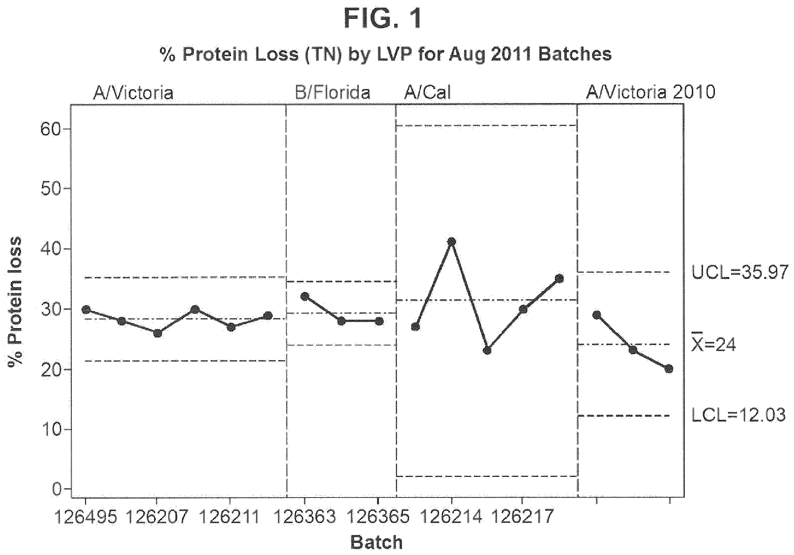

Preliminary mass balance determinations performed on this stage of the process have shown that typically around 30% of the protein in the original material subjected to this filtration is not recovered. Figure 1 shows the losses observed for a number of batches, across three different influenza virus strains: ANictoria (2010), B/Florida and A/California.

The protein lost was confirmed to be virus using a quantitative PCR assay specific for influenza virus RNA. All samples were treated with an RNAase enzyme prior to commencing qPCR analysis to eliminate the detection of non-viral RNA. SDS-PAGE analysis of pre- and post-filtration material also confirmed that whole virus proteins were being removed from the process.

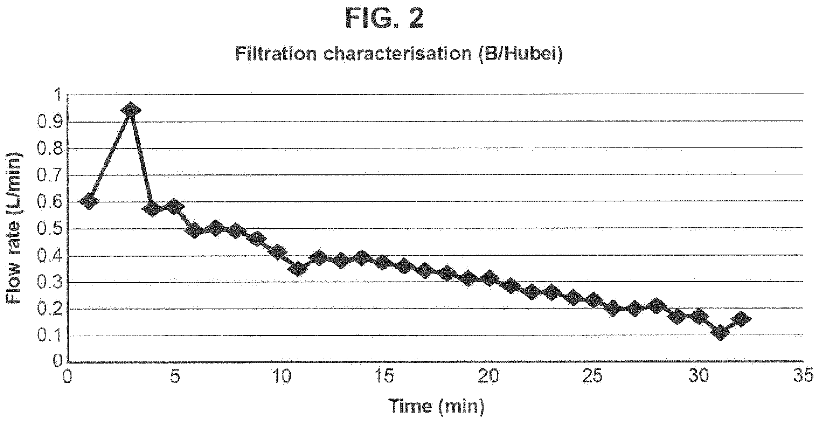

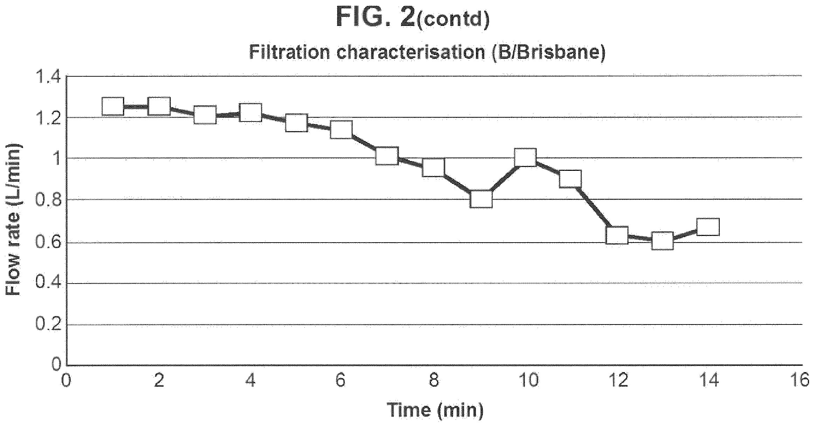

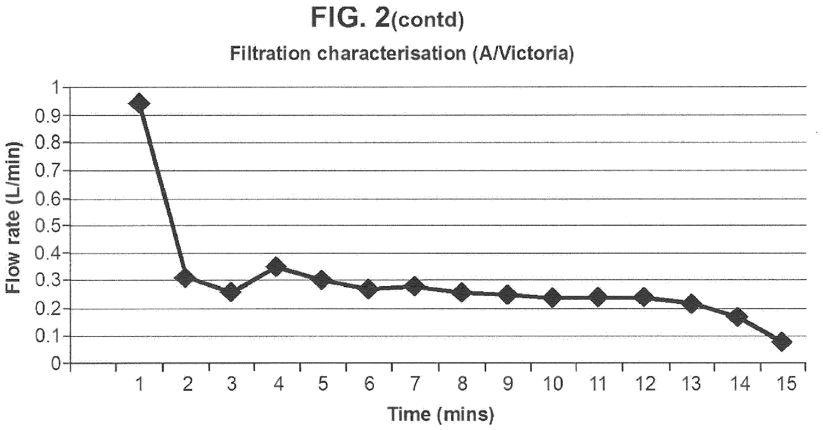

For the three strains evaluated by qPCR, filter blockage was observed during processing. This is indicated by a gradual decrease in flow rate over the course of processing (Figure 2). In each example, 18 litres of product was pumped at a set flowrate of -1.9 litres/min through the filtration train (Sartopure PP2 5 microns/1.2 microns). The filters were blocked after processing between 10-13 litres of the 18 litres volume. At this point a new filter train was used.

The loss of significant amounts of product through these filters and their subsequent blockage was not anticipated. To further characterize the product, pre- and post-filtration samples were analysed by CPS Disc Centrifuge particle sizing procedures.

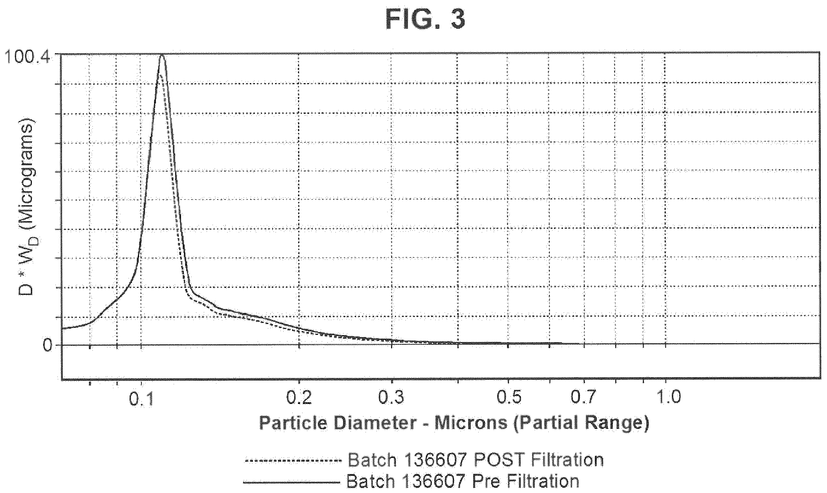

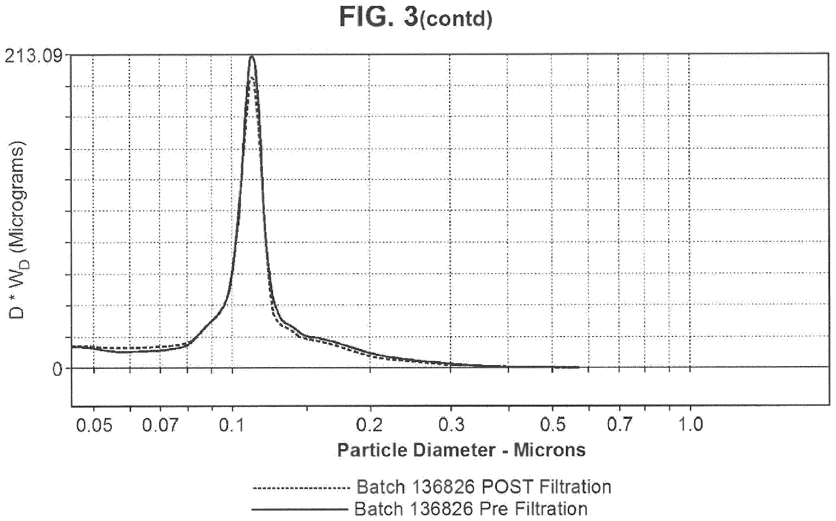

CPS particles sizing profiles for B/Brisbane batches are shown in Figure 3. Both pre- and post-filtration samples were analysed with no difference in size distribution profile observed.

These findings were replicated with an A/Victoria (2010) influenza virus strain; in no instance were significant amounts of particles greater than 1.2 micron in diameter detected before filtration.

As the material which was prevented from passing through the filter was smaller than the pore size, it can be concluded that the material may be binding to the filter matrix and potentially blinding the pores. Filters used for clarification of B/Brisbane were dismantled after use and stained for protein with Coomassie blue dye and compared with a control. Intensive staining was observed on both the 5 and 1.2 micron filters, which was not observed on the control.

Attempts were made to remove the bound protein using acetonitrile, hexane, ethyl acetate, water, chloroform and saline. The protein (whole virus) was irreversibly bound and could not be recovered.

Filter samples either flushed with 300ml of PBS or 300ml of 0.6% w/v polysorbate 80 were dipped into 100ml of pre-filtration sample for 10 mins, removed and stained with Coomassie blue as previously. These results demonstrated protein adsorption to the filter material occurs rapidly in the absence of physically pumping product across the filter. These results also demonstrate that the level of protein adsorption was significantly reduced if the filter surface is treated with polysorbate 80.

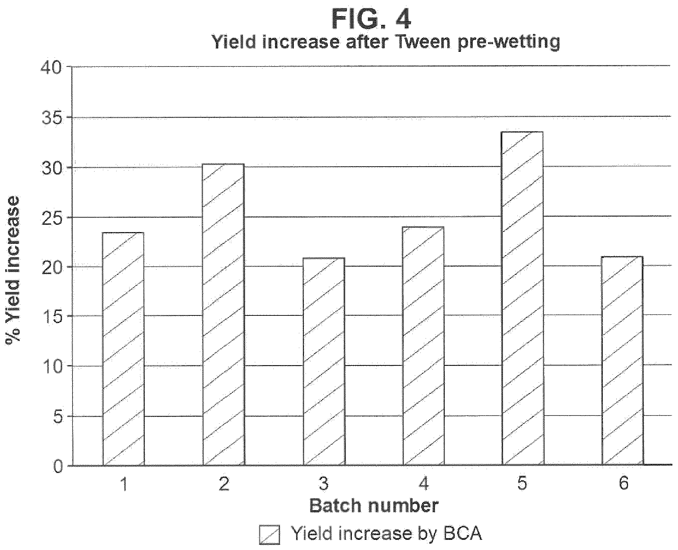

A number of batches of purified influenza virus were each divided into two and filtered with either a standard polypropylene filter, or one that had been previously conditioned (pre-wetted) with polysorbate 80. Increased recoveries of virus, as detected by the BCA assay for protein, were observed for all six batches with the conditioned filter, the improvement ranging from 21-33%, as shown by Figure 4.

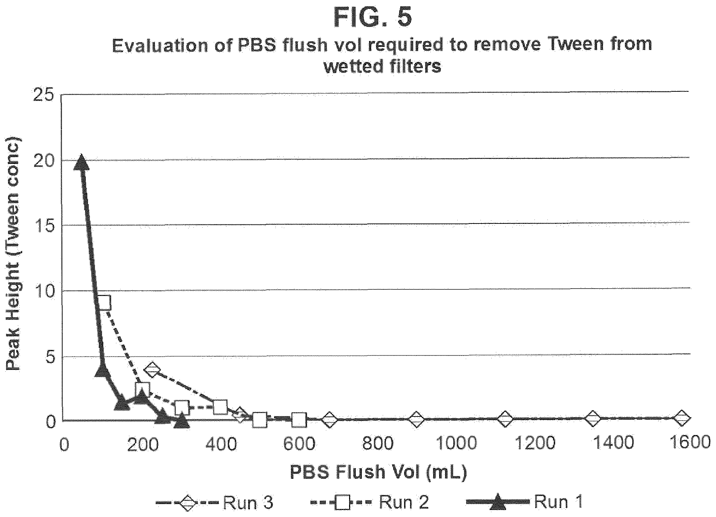

In order to achieve the improved filtration characteristics of the influenza virus, it is not necessary for the polysorbate 80 surfactant to be added to the product. Polysorbate 80 can be allowed to bind to the filter, and free unbound surfactant removed by flushing with phosphate buffered saline (PBS) before passing the product through the filter at a later stage. The flush volume required to remove the unbound surfactant is illustrated in Figure 5.

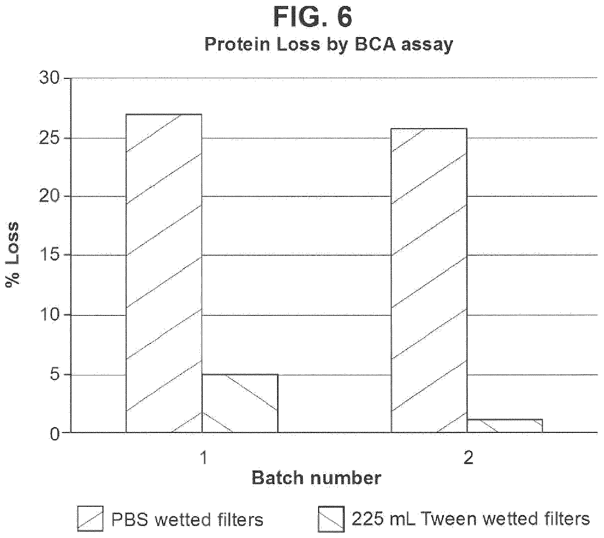

An additional study compared the relative loss of virus that occurred using either a PBS wetted filter or a polysorbate 80 wetted filter. Again, a significant improvement in virus recovery was observed with the polysorbate 80 pre-conditioning (batch number 1 in Figure 6). The virus material that had successfully passed through the polysorbate 80 pre-conditioned filter was subsequently divided into two. One part was passed through a normal PBS wetted filter; the second part was passed through a polysorbate 80 wetted filter. The relative losses are also shown in Figure 6, labelled batch number 2. Negligible loss was detected for the polysorbate 80 wetted filter, whilst 26% of the virus was lost on the PBS wetted filter. Therefore a significant proportion of the virus that had successfully passed through a polysorbate 80 wetted filter was found to subsequently bind to a standard non-treated filter.

The invention has been described by reference to specific examples. However, the application should not be considered as limited to these examples. The scope of the application is defined by the following claims.

The various features and embodiments of the present invention, referred to in individual sections above apply, as appropriate, to other sections, mutatis mutandis. Consequently features specified in one section may be combined with features specified in other sections, as appropriate. However, the present invention is defined by the claims.

Those skilled in the art will recognize, or be able to ascertain using no more than routine experimentation, many equivalents to the specific embodiments of the invention described herein. Such equivalents are intended to be encompassed by the following claims. In particular, the present invention is defined by the claims.

## Claims

1. A method for removing large protein aggregate impurities from a biological product of interest, the method comprising steps of:
pretreating a hydrophobic membrane filter with a surfactant solution having a mass concentration in the range of 0.01 to 10 %w/v surfactant so as to provide a pretreated membrane filter; and,
filtering a fluid comprising a biological product through the pretreated membrane filter so as to obtain a filtrate comprising the biological product;
wherein the membrane filter has a pore size sufficient to retain large protein aggregate impurities present in the fluid; and,
wherein a higher yield of recovery of the biological product in the filtrate is achieved as compared to a non-pretreated control;
wherein the biological product is or comprises a protein, a virus-like particle, a viral particle, a virion, or any combination thereof.

2. A method for preventing loss of a biological product during filtration, the method comprising steps of:
pretreating a hydrophobic membrane filter with a surfactant solution so as to provide a pretreated membrane filter;
wherein the surfactant solution has a mass concentration in the range of 0.01 to 10 %w/v surfactant;
optionally washing the pretreated membrane filter; and,
filtering a fluid comprising large protein aggregate impurities and a first amount of a biological product through the pretreated membrane filter so as to recover the biological product in a filtrate and remove the large protein aggregate impurities, wherein the filtrate comprises a second amount of the biological product;
wherein the biological product is or comprises a protein, a virus-like particle, a viral particle, a virion, or any combination thereof.

3. The method of claim 2, wherein the second amount of the biological product is at least 75% of the first amount of the biological product.

4. A method for filtering a fluid through a pretreated membrane filter, the method comprising the steps of:
(i) providing a fluid sample comprising virus grown in eggs or in a cell culture, wherein the fluid comprises large protein aggregate impurities;
(ii) pretreating a hydrophobic membrane filter with a surfactant solution so as to provide a pretreated membrane filter; wherein the surfactant solution has a mass concentration in the range of 0.01 to 10 %w/v surfactant;
(iii) filtering the fluid sample through the pretreated hydrophobic membrane filter; wherein filtering the fluid removes the large protein aggregate impurities;
(iv) processing the virus into a vaccine formulation.

5. The method of claim 4, wherein the virus is an influenza virus.

6. The method of claim 4 wherein the fluid sample is inactivated prior to filtering.

7. The method of claim 4 further comprising purifying the virus prior to or after the filtering step (ii).

8. The method of claim 4 wherein the purifying step is a depth filtration.

9. The method of claim 4 further comprising splitting the virus with a splitting agent prior to or after the filtering step (ii).

10. A method according to any preceding claim, wherein the surfactant is polysorbate 80.

11. A method according to any preceding claim, wherein the filter comprises polypropylene.

12. A method according to any preceding claim, wherein the pore size of the filter is in the range of 0.1 µm to 10 µm.

13. A method according to any preceding claim, wherein the protein is an influenza surface protein.

14. A method according to any preceding claim, wherein the fluid is additionally filtered through a second treated filter according to any preceding claim to produce the filtrate.

## Patentansprüche

1. Verfahren zum Entfernen von großen Proteinaggregatverunreinigungen aus einem biologischen Produkt von Interesse, wobei das Verfahren die folgenden Schritte umfasst:
die Vorbehandlung eines hydrophoben Membranfilters mit einer Tensidlösung mit einer Massenkonzentration im Bereich von 0,01 bis 10% w/v Tensid unter Bereitstellung eines vorbehandelten Membranfilters und
das Filtrieren einer ein biologisches Produkt umfassenden Flüssigkeit über den vorbehandelten Membranfilter unter Erhalt eines das biologische Produkt umfassenden Filtrats,
wobei der Membranfilter eine Porengröße aufweist, die ausreicht, um in der Flüssigkeit vorhandene große Proteinaggregatverunreinigungen zurückzuhalten und
wobei im Vergleich zu einer nicht behandelten Kontrolle eine höhere Ausbeute an biologischem Produkt in dem Filtrat erzielt wird,
wobei es sich bei dem biologischen Produkt um ein Protein, ein virusähnliches Partikel, ein virales Partikel, ein Virion oder eine beliebige Kombination davon handelt oder das biologische Produkt ein Protein, ein virusähnliches Partikel, ein virales Partikel, ein Virion oder eine beliebige Kombination davon umfasst.

2. Verfahren zum Verhindern des Verlusts eines biologischen Produkts während einer Filtration, wobei das Verfahren die folgenden Schritte umfasst:
die Vorbehandlung eines hydrophoben Membranfilters mit einer Tensidlösung unter Bereitstellung eines vorbehandelten Membranfilters,
wobei die Tensidlösung eine Massenkonzentration im Bereich von 0,01 bis 10% w/v Tensid aufweist,
gegebenenfalls Waschen des vorbehandelten Membranfilters und
das Filtrieren einer große Proteinaggregatverunreinigungen und eine erste Menge eines biologischen Produkts umfassenden Flüssigkeit über den vorbehandelten Membranfilter zur Wiedergewinnung des biologischen Produkts in einem Filtrat und zum Entfernen der großen Proteinaggregatverunreinigungen, wobei das Filtrat eine zweite Menge des biologischen Produkts umfasst,
wobei es sich bei dem biologischen Produkt um ein Protein, ein virusähnliches Partikel, ein virales Partikel, ein Virion oder eine beliebige Kombination davon handelt oder das biologische Produkt ein Protein, ein virusähnliches Partikel, ein virales Partikel, ein Virion oder eine beliebige Kombination davon umfasst.

3. Verfahren nach Anspruch 2, wobei die zweite Menge des biologischen Produkts mindestens 75% der ersten Menge des biologischen Produkts beträgt.

4. Verfahren zum Filtrieren einer Flüssigkeit über einen vorbehandelten Membranfilter, wobei das Verfahren die folgenden Schritte umfasst:
(i) die Bereitstellung einer Flüssigkeitsprobe, die in Eiern oder in einer Zellkultur kultiviertes Virus umfasst, wobei die Flüssigkeit große Proteinaggregatverunreinigungen umfasst,
(ii) die Vorbehandlung eines hydrophoben Membranfilters mit einer Tensidlösung unter Bereitstellung eines vorbehandelten Membranfilters, wobei die Tensidlösung eine Massenkonzentration im Bereich von 0,01 bis 10% w/v Tensid aufweist,
(iii) das Filtrieren der Flüssigkeitsprobe über den vorbehandelten hydrophoben Membranfilter, wobei durch das Filtrieren der Flüssigkeit die großen Proteinaggregatverunreinigungen entfernt werden,
(iv) das Verarbeiten des Virus zu einer Impfstoffformulierung.

5. Verfahren nach Anspruch 4, wobei es sich bei dem Virus um ein Influenzavirus handelt.

6. Verfahren nach Anspruch 4, wobei die Flüssigkeitsprobe vor dem Filtrieren inaktiviert wird.

7. Verfahren nach Anspruch 4, weiterhin umfassend die Aufreinigung des Virus vor oder nach dem Filtrierschritt (ii) .

8. Verfahren nach Anspruch 4, wobei es sich bei dem Aufreinigungsschritt um eine Tiefenfiltration handelt.

9. Verfahren nach Anspruch 4, weiterhin umfassend das Splitting des Virus mit einem Splittingmittel vor oder nach dem Filtrierschritt (ii).

10. Verfahren nach einem der vorhergehenden Ansprüche, wobei es sich bei dem Tensid um Polysorbat 80 handelt.

11. Verfahren nach einem der vorhergehenden Ansprüche, wobei der Filter Polypropylen umfasst.

12. Verfahren nach einem der vorhergehenden Ansprüche, wobei die Porengröße des Filters im Bereich von 0,1 µm bis 10 µm liegt.

13. Verfahren nach einem der vorhergehenden Ansprüche, wobei es sich bei dem Protein um ein Influenza-Oberflächenprotein handelt.

14. Verfahren nach einem der vorhergehenden Ansprüche, bei dem man die Flüssigkeit zusätzlich über einen zweiten behandelten Filter nach einem der vorhergehenden Ansprüche filtriert, unter Bildung des Filtrats.

## Revendications

1. Méthode destinée à éliminer des impuretés d'agrégats protéiques de grande taille à partir d'un produit biologique d'intérêt, la méthode comprenant les étapes consistant à :
prétraiter un filtre à membrane hydrophobe par une solution d'agent tensioactif ayant une concentration massique dans la plage allant de 0,01 à 10% p/v d'agent tensioactif, de façon à fournir un filtre à membrane prétraité ; et
filtrer un fluide comprenant un produit biologique sur le filtre à membrane prétraité, de façon à obtenir un filtrat comprenant le produit biologique ;
où le filtre à membrane possède une taille de pores suffisante pour retenir les impuretés d'agrégats protéiques de grande taille présentes dans le fluide ; et
où un rendement supérieur de récupération du produit biologique dans le filtrat est obtenu, par rapport à un témoin non prétraité ;
où le produit biologique est ou comprend une protéine, une particule pseudo-virale, une particule virale, un virion, ou une combinaison quelconque de ceux-ci.

2. Méthode destinée à empêcher la perte d'un produit biologique lors de la filtration, la méthode comprenant les étapes consistant à :
prétraiter un filtre à membrane hydrophobe par une solution d'agent tensioactif de façon à fournir un filtre à membrane prétraité ;
où la solution d'agent tensioactif possède une concentration massique dans la plage allant de 0,01 à 10% p/v d'agent tensioactif ;
éventuellement laver le filtre à membrane prétraité ; et filtrer un fluide comprenant des impuretés d'agrégats protéiques de grande taille et une première quantité d'un produit biologique sur le filtre à membrane prétraité,
de façon à récupérer le produit biologique dans un filtrat et éliminer les impuretés d'agrégats protéiques de grande taille, où le filtrat comprend une deuxième quantité du produit biologique ;
où le produit biologique est ou comprend une protéine, une particule pseudo-virale, une particule virale, un virion, ou une combinaison quelconque de ceux-ci.

3. Méthode selon la revendication 2, dans laquelle la deuxième quantité du produit biologique constitue au moins 75% de la première quantité du produit biologique.

4. Méthode destinée à filtrer un fluide sur un filtre à membrane prétraité, la méthode comprenant les étapes consistant à :
(i) mettre à disposition un échantillon de fluide comprenant un virus cultivé dans des œufs ou dans une culture cellulaire, le fluide comprenant des impuretés d'agrégats protéiques de grande taille ;
(ii) prétraiter un filtre à membrane hydrophobe par une solution d'agent tensioactif de façon à fournir un filtre à membrane prétraité ; où la solution d'agent tensioactif possède une concentration massique dans la plage allant de 0,01 à 10% p/v d'agent tensioactif ;
(iii) filtrer l'échantillon de fluide sur le filtre à membrane hydrophobe prétraité ; où la filtration du fluide élimine les impuretés d'agrégats protéiques de grande taille ;
(iv) mettre le virus sous forme de formulation de vaccin.

5. Méthode selon la revendication 4, dans laquelle le virus est un influenzavirus.

6. Méthode selon la revendication 4, dans laquelle l'échantillon de fluide est inactivé préalablement à la filtration.

7. Méthode selon la revendication 4, comprenant en outre la purification du virus avant ou après l'étape de filtration (ii).

8. Méthode selon la revendication 4, dans laquelle l'étape de purification est une filtration en profondeur.

9. Méthode selon la revendication 4, comprenant en outre la dissociation du virus à l'aide d'un agent de dissociation avant ou après l'étape de filtration (ii).

10. Méthode selon l'une quelconque des revendications précédentes, dans laquelle l'agent tensioactif est le polysorbate 80.

11. Méthode selon l'une quelconque des revendications précédentes, dans laquelle le filtre comprend du polypropylène.

12. Méthode selon l'une quelconque des revendications précédentes, dans laquelle la taille de pores du filtre se trouve dans la plage allant de 0,1 µm à 10 µm.

13. Méthode selon l'une quelconque des revendications précédentes, dans laquelle la protéine est une protéine de surface grippale.

14. Méthode selon l'une quelconque des revendications précédentes, dans laquelle le fluide est en outre filtré sur un deuxième filtre traité selon l'une quelconque des revendications précédentes, afin de produire le filtrat.
